# EUROPEAN PATENT SPECIFICATION

(11) **EP 3 563 381 B1**
(45) Date of publication and mention of the grant of the patent: **07.02.2024**
(21) Application number: 17822683.3
(22) Date of filing: 21.12.2017
(51) Int. Cl.: G16H 40/63

(54) **SYSTEM AND METHOD FOR ANALYZING THE USAGE OF PORTABLE MEDICAL DEVICES AND IMPROVING THE UTILIZATION OF SUCH DEVICES**
SYSTEM UND VERFAHREN ZUR ANALYSE DER VERWENDUNG TRAGBARER MEDIZINISCHER VORRICHTUNGEN UND ZUR VERBESSERUNG DER VERWENDUNG SOLCHER VORRICHTUNGEN
SYSTÈME ET PROCÉDÉ D'ANALYSE DE L'UTILISATION DE DISPOSITIFS MÉDICAUX PORTABLES ET D'AMÉLIORATION DE L'UTILISATION DE TELS DISPOSITIFS

(30) Priority: 29.12.2016 US 201662440019 P
(43) Date of publication of application: 06.11.2019
(73) Proprietor: Koninklijke Philips N.V., 5656 AG Eindhoven (NL)
(72) Inventor: CYRAN, Robert, John, 5656 AE Eindhoven (NL)
(74) Representative: Philips Intellectual Property & Standards
(86) International application number: PCT/EP2017/084274
(87) International publication number: WO 2018/122135

(56) References cited:
- US-A1- 2002 188 173
- US-A1- 2010 141 744
- US-A1- 2015 230 695

## Description

### BACKGROUND OF THE INVENTION

### 1. Field of the Invention

The present invention relates to systems for analyzing the usage of portable medical devices and improving the utilization of such devices. The present invention also relates to methods for analyzing the usage of portable medical devices and improving the utilization of such devices.

### 2. Description of the Related Art

Portable medical devices, such as those used to perform Home Sleep Tests (HSTs), pass through a number of different states in their normal usage cycle. For example, the following set of states could be used to define the usage cycle of an HST device:
I. Available (i.e. can be scheduled for an HST)
II. Configured (e.g. patient information, information that specifies which signals to record, etc. downloaded to the device in preparation of an HST)
III. Delivered to patient
IV. Returned by patient
V. Cleaning
VI. Recharging batteries

Conventional scheduling approaches focus simply on whether a device is "Available" to be given to a patient for use or alternatively is "Unavailable" to be given to a patient for use. However, device owners (e.g. sleep labs, DMEs, hospitals) could better manage their inventory if they knew the amount of time spent in each state. For example, if the percentage of time spent in the "Available" state is very low, this could indicate that additional devices need to be purchased to increase the inventory. On the other hand, if the percentage of time spent in the "Available" state is high, this could indicate an excess of devices in the inventory. Statistics that report the amount of time spent in the other states can help device owners recognize potential inefficiencies in their process. For example, they may see that it is taking a long time before patients return the device, or it is taking too long to clean the devices after they are returned. Appropriate actions could then be taken to improve these situations and shorten the usage cycle. Shortening the usage cycle allows devices to be rescheduled for additional HSTs sooner. Over time, shorter usage cycles lead to a larger number of patients having HSTs performed on them without having to increase the number of devices in the inventory.

In the absence of usage cycle statistics, the scheduling of devices requires estimating how long a usage cycle will take. Specifically, it requires estimating the duration between when a device first becomes unavailable until it becomes available again. In this example, this is the duration from the time the device enters the "Configured" state until the time the batteries have been recharged. If this duration is estimated to take a week, then a device cannot be scheduled for another HST until a week after the start of state 2 in the device's current usage cycle. Furthermore, to ensure that devices are actually available before they are scheduled for use, these estimates are usually conservative. Otherwise, if the estimates are too aggressive, a device may not be available by the date it is next scheduled, which would result in a cancelation of the HST that is to be performed on the next patient.

The conservative estimates can result in underutilization of the devices if those devices are actually in the "Available" state for significant periods of time. Accordingly,

Document US 2015/230695 A1 discloses a control method for a gastrointestinal tract diagnosis system that based on a usage time of the endoscope.

Document US 2010/141744 A1 discloses a system for wirelessly powering various devices positioned on an endoscope.

### SUMMARY OF THE INVENTION

The invention is defined by the claims.

The invention comprises a computer-implemented method for improving utilization of portable medical devices by a practitioner, the method comprising: determining a plurality of device states within a test cycle that are to be monitored, at least one of the device states corresponding to when a portable medical device is in the possession of a patient and at least two of said device states corresponding to when the portable medical device is in the possession of the practitioner; monitoring the time one or more portable medical devices are in one of the device states; determining that the time spent in the one device state by one portable medical device or the average time spent in the one device state by more than one portable medical device is greater than an optimum time; and wherein the plurality of device states comprise one or more of: a state corresponding to the portable medical device undergoing a recharging process; and a state corresponding to the portable medical device undergoing a cleaning process, and wherein the method further comprises, responsive to determining that the time, or the average time, spent in the state corresponding to the portable medical device undergoing one or more of a recharging process and a cleaning process by one or more than one portable medical device is greater than an optimum time, determining that an inefficiency exists in the usage of said one or more than one portable medical device, providing an indication if the time spent is greater than the optimum time.

Determining a plurality of device states within a test cycle that are to be monitored may comprise receiving one or more indications from a practitioner of the device states that are to be monitored.

At least one device state may correspond to the portable medical device being delivered to the patient.

At least two device states may correspond to the portable medical device being available and to the portable medical device being configured to be used by a particular patient.

Providing an indication that the time spent in the particular state by the one portable medical device or the average time spent in the particular state by the more than one portable medical device is greater than the optimum time may comprise providing an indication on a display.

Monitoring the time one or more portable medical devices are in one of the device states may comprise storing the time spent in the one device state in a memory associated with a processing device.

The optimum time may comprise a time value received from the practitioner.

The optimum time may comprise a time value determined from a plurality of other test cycles.

Providing an indication that the time spent in the one device state by the one portable medical device or the average time spent in the one device state by the more than one portable medical device is greater than the optimum time may comprise providing an indication of a corrective action.

According to another aspect of the present invention, such improvement is provided by a system comprising: a processing device having a suitable memory associated therewith; and an input/out device structured to receive information from, and provide information to, a user, wherein the processing device is programmed to: determine a plurality of device states within a test cycle that are to be monitored, at least one of the device states corresponding to when a portable medical device is in the possession of a patient and at least two of said device states corresponding to when the portable medical device is in the possession of the practitioner; monitor the time one or more portable medical devices are in one of the device states; determine that the time spent in the one device state by one portable medical device or the average time spent in the one device state by more than one portable medical device is greater than an optimum time; and wherein the plurality of device states comprise one or more of: a state corresponding to the portable medical device undergoing a recharging process; and a state corresponding to the portable medical device undergoing a cleaning process, and wherein the method further comprises, responsive to determining that the time, or the average time, spent in the state corresponding to the portable medical device undergoing one or more of a recharging process and a cleaning process by one or more than one portable medical device is greater than an optimum time, determining that an inefficiency exists in the usage of said one or more than one portable medical device and providing an indication if the time spent is greater than the optimum time.

The processor may be programmed to determine the plurality of device states within a test cycle that are to be monitored at least in-part from input received from the input/output device.

The indication that the time spent in the one device state by the one portable medical device or the average time spent in the one device state by the more than one portable medical device is greater than the optimum time may be provided in a display on the input/output device.

The processor may be further programmed to provide an indication of a corrective action.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a flow chart of a method in accordance with an example embodiment of the present invention;
FIG. 2 is a system in accordance with an example embodiment of the present invention upon which the method of FIG. 1 may be carried out;
FIG. 3 is an input screen in accordance with an example embodiment of the present invention;
FIG. 4 is an output screen in accordance with an example embodiment of the present invention; and
FIG. 5 is an output screen in accordance with another example embodiment of the present invention.

### DETAILED DESCRIPTION OF THE INVENTION

Embodiments of the present invention provide owners of portable devices insight into the usage of their devices. Owners can analyze the usage cycle statistics that are automatically computed by the system to determine inefficiencies that may exist in certain steps of that usage cycle. Action can then be taken to address the identified inefficiencies.

As used herein, the singular form of "a", "an", and "the" include plural references unless the context clearly dictates otherwise.

As used herein, the term "number" shall mean one or an integer greater than one (i.e., a plurality).

As used herein, "device" shall be used to an apparatus which is tracked as it passes through a plurality of states in a testing cycle.

As used herein, "state" shall be used to refer to one or both of a location or current useage of a device at a particular time within a test cycle.

As used herein, "test cycle" or "testing cycle" shall refer to a predetermined series of states through which a device passes before, during, and after a testing session with a patient.

As used herein, "user" or "practitioner" refers to any person or persons who utilize and interact with a system in accordance with the present invention in order to analyze and optimize usage of medical devices throughout a testing cycle.

As used herein, "patient" refers to the person to which a device is configured who will be the subject studied during a particular test cycle.

As used herein, the term "controller" or "processing device" shall mean a programmable analog and/or digital device (including an associated memory part or portion) that can store, retrieve, execute and process data (e.g., software routines and/or information used by such routines), including, without limitation, a field programmable gate array (FPGA), a complex programmable logic device (CPLD), a programmable system on a chip (PSOC), an application specific integrated circuit (ASIC), a microprocessor, a microcontroller, a programmable logic controller, or any other suitable processing device or apparatus. The memory portion can be any one or more of a variety of types of internal and/or external storage media such as, without limitation, RAM, ROM, EPROM(s), EEPROM(s), FLASH, and the like that provide a storage register, i.e., a non-transitory machine readable medium, for data and program code storage such as in the fashion of an internal storage area of a computer, and can be volatile memory or nonvolatile memory.

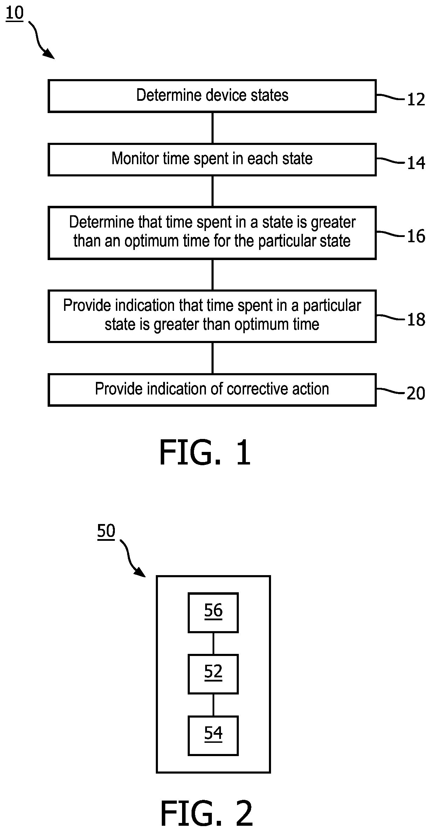

Figure 1 shows a flow chart of a method 10 for analyzing the usage of portable medical devices and improving the utilization of such devices in accordance with an example embodiment of the present invention. Figure 2 shows an example system 50 which may be used to carry out the method of Figure 1. System 50 includes a processing device 52 having a suitable memory 54 associated therewith. System 50 also includes an input/output device 56 in communication with processing device 52 generally for providing input to, and/or receiving output from, processing device 52. In example embodiments of the present invention, system 50 is a smartphone or tablet and input/output device 56 is a touchscreen. In another example embodiment, system 50 is a computer and input/output device 56 is a separate keyboard and display.

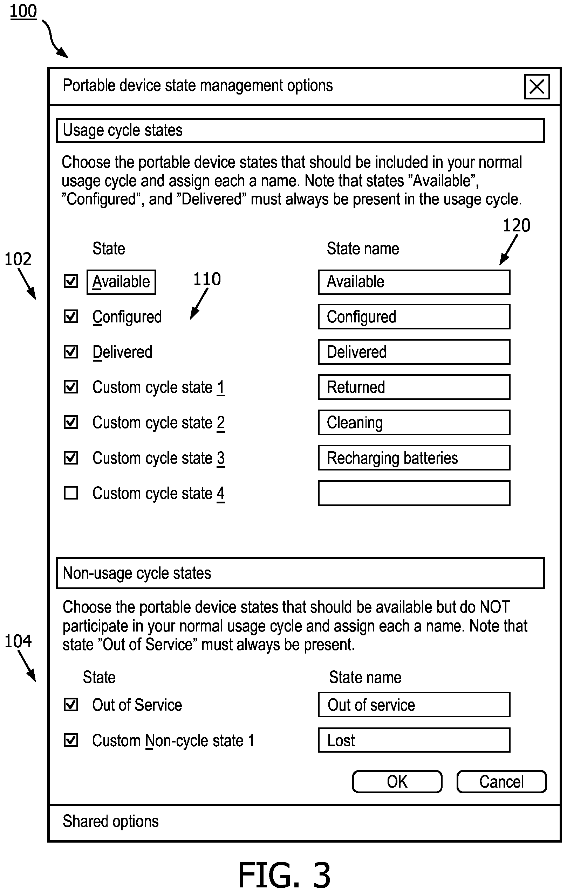

Referring again to Figure 1, method 10 begins with an initial step 12 where it is determined what different device states a test cycle for a portable medical device (e.g., an HST device) will be divided into. Some examples of such states have been previously described in the background section. Such determination may be made by prompting a user of the system (i.e., a practitioner) to identify the different states in which devices which are to be monitored may be classified at various times in a test cycle. Figure 3 shows an example input display 100 which may be provided on input/output device 56 in carrying out step 12 for prompting a user for input and receiving indications from the user in accordance with an example embodiment of the present invention. In the example shown in Figure 3, input screen 100 includes a first section 102 directed to "Usage Cycle States" (i.e., states in which a device would be classified during a typical test cycle) and a second section 104 directed to "Non-Usage Cycle States" (i.e., states in which a device might be classified which are not part of a typical test cycle). In first section 102, the user is provided with a number of states, generally indicated at 110, which may be selected/identified (e.g., without limitation, via check boxes or other suitable means) by the user. A custom state name, shown generally by 120, may be provided by the user to identify each of the states selected/identified by the user. In an example embodiment of the present invention, such as that shown in Figure 3, the "Available", "Configured" and "Delivered" states are defined (i.e., the user cannot uncheck these states) as such states represent the three basic steps that must be present. An "Out of Service" state is also defined in the non-usage cycle set. These particular states are always present in such embodiment as the system includes built-in support that automatically moves devices into and out of these states when certain functions are performed on those devices.

For example, one such function allows users to configure a device (i.e. send configuration information to the device in preparation for a home sleep study). When the configuration operation is performed, the state of the device is automatically set to "Configured". In the example illustrated in Figure 3 the additional usage-cycle states of "Returned", "Cleaning", and "Recharging Batteries" have been added by the user because these states are involved in the normal process flow of their devices, and the user has a desire to track devices that are in these states and compute statistics for these states. In the example of Figure 3, the user has also added an additional non-flow cycle state, which is "Lost" in second section 104. Although the example of Figure 3 allows four additional custom usage-cycle states to be defined as well as one additional non-usage cycle state, it is to be appreciated that such quantities are given for exemplary purposes only and that other quantities may be employed without varying from the scope of the present invention.

After determining the different states 110 into which a test cycle will be divided in step 12, the time spent in each of such states 110 by devices during test cycles is monitored in step 14. Processing device 52 can automatically reassign a device to a specific state when certain operations are performed using processing device 52. For example, when a new device is added to system 50 (i.e., to memory 54), the state of that device is automatically set to "Available" as such new device typically would be ready for use as soon as it is added. When a user configures a device (i.e. downloads patient information, information that specifies which signals to record, etc. to the device), the state of that device is automatically set to "Configured". When the sleep study is retrieved from the device, the device state is automatically set to "Returned" as such retrieval would only be carried out once a device has been returned by a patient. Alternatively, the system also provides an interface that allows users to manually set the state of a device to any one of the available states. This interface can be used to move a device to a custom state or a non-usage cycle state.

Generally at any point after the device monitoring of step 14 commences, it may be determined by processing device 52 that the time spent by a device, or the time spent by several devices, in a given state is greater than a predetermined optimum time for such state, such as shown in step 16. Such optimum time may be set by a user (e.g., in configuration options), set by a manufacturer, determined from analysis of other test cycles (e.g., where the number of test cycles is sufficient so that optimum duration for each state can be computed with a high degree of confidence (i.e. so the determination of an optimum duration for a particular state is not skewed by a small number of durations that were very short or very long), determined from analysis of other test cycles carried out by another completely distinct user with distinct devices from the present user, or from any other suitable source. If such determination at step 16 occurs, then an indication is provided to the user (such a via input/output device 56, discussed further below) that an apparent inefficiency exists that should be addressed, such as shown in step 18. As an example, if a manufacturer indicates that the time for charging a device should be 3 hours and monitoring during step 14 determines it is taking 3.5 hours for a particular device, or an average of 3.3 hours for several devices, then an indication is provided to the user that something needs addressed. In the case of a charging state taking longer than anticipated, such delay could be a result of improper charging, faulty batteries, operator error, etc.

By performing statistical analysis on the data obtained in step 14, a likely corrective action can also be determined which addresses the inefficiency determined in step 16 and, as shown in step 20, an indication of such corrective action which should be taken can be provided to the user. For example, if the average cleaning time for several devices is 6 hours, but for one particular device it is 7.4 hours, it could be concluded that the particular device likely has a flaw or flaws which are requiring additional cleaning time and thus perhaps should be removed from test cycling.

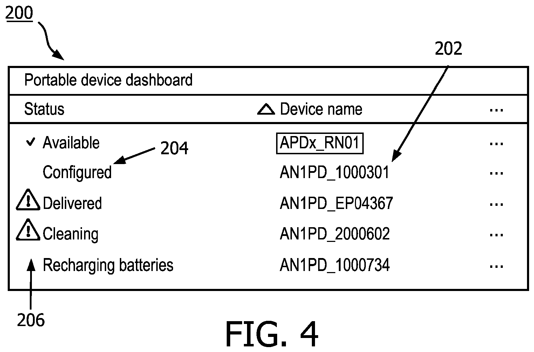

Figure 4 shows an example output display 200 which may be employed in carrying out steps 18 and/or 20 in providing indications to a user regarding real-time performance of devices in test cycles in accordance with an example embodiment of the present invention. Display 200 includes a listing of devices 202 which are being monitored as well as the present state 204 in which each device is presently disposed. Display 200 also includes indications 206, such as may be provided in step 18, in accordance with an example embodiment of the invention. In such example, it was determined (such as previously discussed in regard to step 16) that device "AN1PD_EP04367" has been in the "Delivered" state for longer than an optimum time, and also that device "AN1PD_2000602" has been in the "Cleaning" state for longer than an optimum time. By receiving such "real-time" feedback, potential issues can be addressed immediately by a user.

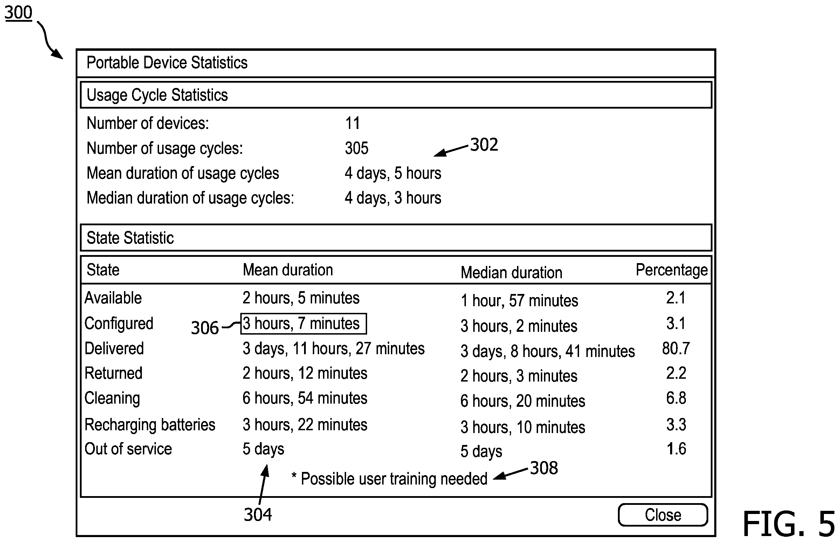

Figure 5 shows an example output display 300 which may be employed in carrying out steps 18 and/or 20 in providing information/indications to a user regarding overall performance of test cycles in accordance with another example embodiment of the present invention. Display 300 include a first section 302 directed to "Usage Cycle Statistics" which displays various statistics (e.g., without limitation, number of devices, number of usage cycles, mean duration, median duration, etc.) for the overall test cycles that have been monitored for a selected period of time. Display 300 also includes a second section 304 directed to "State Statistics" which displays various statistics (e.g., without limitation, mean duration, median duration, percentage, etc.) for one or more of the states which have been monitored.

Figure 5 also provides another example of an indication 306, such as may be provided in step 18, in accordance with an example embodiment of the invention. In such example, the mean time for the "Configured" state was determined to be more than the predetermined optimum value and thus has been particularly singled out for attention by the user. Figure 5 also provides an example of yet another indication 308, such as may be provided in step 20, of a suggested corrective action to take in order to address the inefficiency identified by indication 306. It is to be appreciated that indications 306 and 308 are given for example purposes only and that other suitable indications may be employed without varying from the scope of the present invention.

In addition to carrying out the method previous described, processing device 52 may include a scheduler that can be used to schedule devices, such as for use in HSTs. In order to schedule devices, the scheduler must know the duration of a usage cycle, otherwise, it cannot know when a device that is currently in-use will become available. Initially, the scheduler will use a usage cycle duration value that has been specified in a configuration options section. This value is a "best guess" estimate, normally padded to ensure the scheduling does not result in schedules that cannot be met. The scheduler will continue to use the usage cycle duration value specified in the options until there are a sufficient number of usage cycles present in a history table, such as would be populated from step 14 previously discussed. More specifically, when the number of usage cycles in the history table is considered large enough to compute average usage cycle durations with a high degree of confidence (i.e. the computation of average usage cycle duration is not skewed by a small number of usage cycles that have very short or long durations), the scheduler will use the average usage cycle duration computed by processing device 52 to determine when devices become available. Over time, as the number of usage cycles in the table continues to grow and exceed certain threshold counts, processing device 52 can begin computing a weighted average for the usage cycle durations instead of a simple average, giving precedence to those usage cycles that were observed more recently.

In summary, the invention thus proposes a system and method that allows a practitioner to monitor the time numerous devices spend in various states during test cycles for each of the devices. Through analysis of the data obtained from such monitoring, inefficiencies in the test cycle can be identified and addressed so as to improve usage of the devices and thus improve the overall efficiency of the test cycles. Additionally, data obtained regarding test cycles can be used to optimize overall scheduling of devices.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method for improving utilization of portable medical devices by a practitioner, the method comprising:
determining a plurality of device states within a test cycle that are to be monitored, at least one of the device states corresponding to when a portable medical device is in the possession of a patient and at least two of said device states corresponding to when the portable medical device is in the possession of the practitioner;
monitoring the time one or more portable medical devices are in one of the device states;
determining that the time spent in the one device state by one portable medical device or the average time spent in the one device state by more than one portable medical device is greater than an optimum time; and
wherein the plurality of device states comprise one or more of:
a state corresponding to the portable medical device undergoing a recharging process; and
a state corresponding to the portable medical device undergoing a cleaning process, and
wherein the method further comprises, responsive to determining that the time, or the average time, spent in the state corresponding to the portable medical device undergoing one or more of a recharging process and a cleaning process by one or more than one portable medical device is greater than an optimum time, determining that an inefficiency exists in the usage of said one or more than one portable medical device, and providing an indication if the time spent is greater than the optimum time.

2. The computer-implemented method of claim 1, wherein determining a plurality of device states within a test cycle that are to be monitored comprises receiving one or more indications from a practitioner of the device states that are to be monitored.

3. The computer-implemented method of claim 1, wherein the at least one device state corresponds to the portable medical device being delivered to the patient.

4. The computer-implemented method of claim 1, wherein the at least two device states correspond to the portable medical device being available and to the portable medical device being configured to be used by a particular patient.

5. The computer-implemented method of claim 1, wherein providing an indication that the time spent in the particular state by the one portable medical device or the average time spent in the particular state by the more than one portable medical device is greater than the optimum time comprises providing an indication on a display.

6. The computer-implemented method of claim 1, wherein monitoring the time one or more portable medical devices are in one of the device states comprises storing the time spent in the one device state in a memory associated with a processing device.

7. The computer-implemented method of claim 1, wherein the optimum time comprises a time value received from the practitioner.

8. The computer-implemented method of claim 1, wherein the optimum time comprises a time value determined from a plurality of other test cycles.

9. The computer-implemented method of claim 1, wherein providing an indication that the time spent in the one device state by the one portable medical device or the average time spent in the one device state by the more than one portable medical device is greater than the optimum time comprises providing an indication of a corrective action.

10. A system (100) comprising:
a processing device (102) having a suitable memory (104) associated therewith; and
an input/out device (106) structured to receive information from, and provide information to, a user,
wherein the processing device is programmed to:
determine a plurality of device states within a test cycle that are to be monitored, at least one of the device states corresponding to when a portable medical device is in the possession of a patient and at least two of said device states corresponding to when the portable medical device is in the possession of the practitioner;
monitor the time one or more portable medical devices are in one of the device states;
determine that the time spent in the one device state by one portable medical device or the average time spent in the one device state by more than one portable medical device is greater than an optimum time; and
wherein the plurality of device states comprise one or more of:
a state corresponding to the portable medical device undergoing a recharging process; and
a state corresponding to the portable medical device undergoing a cleaning process, and
wherein the method further comprises, responsive to determining that the time, or the average time, spent in the state corresponding to the portable medical device undergoing one or more of a recharging process and a cleaning process by one or more than one portable medical device is greater than an optimum time, determining that an inefficiency exists in the usage of said one or more than one portable medical device, and providing an indication if the time spent is greater than the optimum time.

11. The system of claim 10, wherein the processor is programmed to determine the plurality of device states within a test cycle that are to be monitored at least in-part from input received from the input/output device.

12. The system of claim 10, wherein the indication that the time spent in the one device state by the one portable medical device or the average time spent in the one device state by the more than one portable medical device is greater than the optimum time is provided in a display on the input/output device.

13. The system of claim 10, wherein the processor is further programmed to provide an indication of a corrective action.

## Patentansprüche

1. Ein computerimplementiertes Verfahren zur Verbesserung der Nutzung tragbarer medizinischer Geräte durch einen Arzt, wobei das Verfahren Folgendes umfasst: bestimmen mehrerer Gerätezustände innerhalb eines Testzyklus, die überwacht werden sollen, wobei mindestens einer der Gerätezustände dem Zeitpunkt entspricht, wenn sich ein tragbares medizinisches Gerät im Besitz eines Patienten befindet, und mindestens zwei der Gerätezustände dem Zustand entsprechen, wenn das tragbare medizinische Gerät im Besitz eines Patienten ist das medizinische Gerät befindet sich im Besitz des Arztes; überwachen der Zeit, in der sich ein oder mehrere tragbare medizinische Geräte in einem der Gerätezustände befinden; bestimmen, dass die Zeit, die ein tragbares medizinisches Gerät im Ein-Geräte-Zustand verbringt, oder die durchschnittliche Zeit, die mehr als ein tragbares medizinisches Gerät im Ein-Geräte-Zustand verbringt, größer als eine optimale Zeit ist; und wobei die mehreren Gerätezustände einen oder mehrere der folgenden umfassen: ein Zustand, der dem tragbaren medizinischen Gerät entspricht, das einen Aufladevorgang durchläuft; und ein Zustand, der dem tragbaren medizinischen Gerät entspricht, das einem Reinigungsprozess unterzogen wird, und wobei das Verfahren als Reaktion auf das Bestimmen, dass die Zeit oder die durchschnittliche Zeit, die in dem Zustand verbracht wurde, der dem entspricht, dass das tragbare medizinische Gerät einem oder mehreren von einem Aufladevorgang und einem Reinigungsprozess durch ein oder mehr als ein tragbares medizinisches Gerät unterzogen wird, umfasst länger als eine optimale Zeit, Feststellen, dass eine Ineffizienz bei der Verwendung des einen oder der mehreren tragbaren medizinischen Geräte vorliegt, und Bereitstellen einer Anzeige, wenn die aufgewendete Zeit länger als die optimale Zeit ist.

2. Computerimplementiertes Verfahren nach Anspruch 1, wobei das Bestimmen mehrerer zu überwachender Gerätezustände innerhalb eines Testzyklus das Empfangen einer oder mehrerer Hinweise von einem Praktiker über die zu überwachenden Gerätezustände umfasst.

3. Computerimplementiertes Verfahren nach Anspruch 1, wobei der mindestens eine Gerätezustand der Lieferung des tragbaren medizinischen Geräts an den Patienten entspricht.

4. Computerimplementiertes Verfahren nach Anspruch 1, wobei die mindestens zwei Gerätezustände der Verfügbarkeit des tragbaren medizinischen Geräts und der Konfiguration des tragbaren medizinischen Geräts für die Verwendung durch einen bestimmten Patienten entsprechen.

5. Computerimplementiertes Verfahren nach Anspruch 1, wobei ein Hinweis bereitgestellt wird, dass die von dem einen tragbaren medizinischen Gerät in dem bestimmten Zustand verbrachte Zeit oder die durchschnittliche von mehr als einem tragbaren medizinischen Gerät in dem bestimmten Zustand verbrachte Zeit größer ist als die Die optimale Zeit umfasst die Anzeige auf einem Display.

6. Computerimplementiertes Verfahren nach Anspruch 1, wobei das Überwachen der Zeit, die sich ein oder mehrere tragbare medizinische Geräte in einem der Gerätezustände befinden, das Speichern der in dem einen Gerätezustand verbrachten Zeit in einem Speicher umfasst, der einem Verarbeitungsgerät zugeordnet ist.

7. Computerimplementiertes Verfahren nach Anspruch 1, wobei der optimale Zeitpunkt einen vom Praktiker erhaltenen Zeitwert umfasst.

8. Computerimplementiertes Verfahren nach Anspruch 1, wobei der optimale Zeitpunkt einen aus mehreren anderen Testzyklen ermittelten Zeitwert umfasst.

9. Computerimplementiertes Verfahren nach Anspruch 1, wobei ein Hinweis bereitgestellt wird, dass die Zeit, die das eine tragbare medizinische Gerät in dem einen Gerätezustand verbringt, oder die durchschnittliche Zeit, die mehr als ein tragbares medizinisches Gerät in dem einen Gerätezustand verbringt, größer ist als der optimale Zeitpunkt umfasst, einen Hinweis auf eine Korrekturmaßnahme zu geben.

10. Ein System (100), umfassend: ein Verarbeitungsgerät (102), dem ein geeigneter Speicher (104) zugeordnet ist; und ein Eingabe-/Ausgabegerät (106), das so strukturiert ist, dass es Informationen von einem Benutzer empfängt und ihm Informationen bereitstellt, wobei das Verarbeitungsgerät dazu programmiert ist: bestimmen einer Vielzahl von Gerätezuständen innerhalb eines Testzyklus, die überwacht werden sollen, wobei mindestens einer der Gerätezustände dem Zeitpunkt entspricht, wenn sich ein tragbares medizinisches Gerät im Besitz eines Patienten befindet, und mindestens zwei der Gerätezustände dem Zustand entsprechen, wenn das tragbare medizinische Gerät im Besitz eines Patienten ist das medizinische Gerät befindet sich im Besitz des Arztes; überwachen Sie die Zeit, in der sich ein oder mehrere tragbare medizinische Geräte in einem der Gerätezustände befinden; feststellen, dass die Zeit, die ein tragbares medizinisches Gerät im Ein-Geräte-Zustand verbringt, oder die durchschnittliche Zeit, die mehr als ein tragbares medizinisches Gerät im Ein-Geräte-Zustand verbringt, größer als eine optimale Zeit ist; und wobei die mehreren Gerätezustände einen oder mehrere der folgenden umfassen: ein Zustand, der dem tragbaren medizinischen Gerät entspricht, das einen Aufladevorgang durchläuft; und ein Zustand, der dem tragbaren medizinischen Gerät entspricht, das einem Reinigungsprozess unterzogen wird, und wobei das Verfahren als Reaktion auf das Bestimmen, dass die Zeit oder die durchschnittliche Zeit, die in dem Zustand verbracht wurde, der dem entspricht, dass das tragbare medizinische Gerät einem oder mehreren von einem Aufladevorgang und einem Reinigungsprozess durch ein oder mehr als ein tragbares medizinisches Gerät unterzogen wird, umfasst länger als eine optimale Zeit, Feststellen, dass eine Ineffizienz bei der Verwendung des einen oder mehrerer tragbarer medizinischer Geräte vorliegt, und Bereitstellen einer Anzeige, wenn die aufgewendete Zeit länger als die optimale Zeit ist.

11. System nach Anspruch 10, wobei der Prozessor so programmiert ist, dass er die mehreren Gerätezustände innerhalb eines Testzyklus, die überwacht werden sollen, zumindest teilweise anhand der vom Eingabe-/Ausgabegerät empfangenen Eingabe bestimmt.

12. Das System nach Anspruch 10, wobei die Angabe, dass die Zeit, die das eine tragbare medizinische Gerät in dem einen Gerätezustand verbringt, oder die durchschnittliche Zeit, die das mehr als ein tragbares medizinisches Gerät in dem einen Gerätezustand verbringt, größer ist als die optimale Zeit wird in einer Anzeige auf dem Ein-/Ausgabegerät bereitgestellt.

13. Das System nach Anspruch 10, wobei der Prozessor außerdem so programmiert ist, dass er einen Hinweis auf eine Korrekturmaßnahme liefert.

## Revendications

1. Procédé mis en œuvre par ordinateur pour améliorer l'utilisation de dispositifs médicaux portables par un praticien, le procédé comprenant: déterminer une pluralité d'états de dispositif au cours d'un cycle de test qui doivent être surveillés, au moins un des états de dispositif correspondant au moment où un dispositif médical portable est en possession d'un patient et au moins deux desdits états de dispositif correspondant au moment où le dispositif médical portable est en possession d'un patient le dispositif médical est en possession du praticien; surveiller le temps pendant lequel un ou plusieurs dispositifs médicaux portables se trouvent dans l'un des états de dispositif; déterminer que le temps passé dans l'état d'un dispositif par un dispositif médical portable ou le temps moyen passé dans l'état d'un dispositif par plus d'un dispositif médical portable est supérieur à un temps optimal; et dans lequel la pluralité d'états de dispositif comprend un ou plusieurs des éléments suivants: un état correspondant au dispositif médical portable subissant un processus de recharge; et un état correspondant au dispositif médical portable subissant un processus de nettoyage, et le procédé consistant en outre à déterminer que le temps, ou le temps moyen, passé dans l'état correspondant au dispositif médical portable subissant un ou plusieurs processus de recharge et un processus de nettoyage par un ou plusieurs dispositifs médicaux portables est supérieur à un temps optimal, déterminer qu'il existe une inefficacité dans l'utilisation dudit ou desdits dispositifs médicaux portables, et fournir une indication si le temps passé est supérieur au temps optimal.

2. Procédé mis en œuvre par ordinateur selon la revendication 1, dans lequel la détermination d'une pluralité d'états de dispositif dans un cycle de test qui doivent être surveillés comprend la réception d'une ou plusieurs indications d'un praticien sur les états de dispositif qui doivent être surveillés.

3. Procédé mis en œuvre par ordinateur selon la revendication 1, dans lequel le au moins un état du dispositif correspond au dispositif médical portable délivré au patient.

4. Procédé mis en œuvre par ordinateur selon la revendication 1, dans lequel les au moins deux états de dispositif correspondent au dispositif médical portable disponible et au dispositif médical portable configuré pour être utilisé par un patient particulier.

5. Procédé mis en œuvre par ordinateur selon la revendication 1, dans lequel la fourniture d'une indication selon laquelle le temps passé dans l'état particulier par le dispositif médical portable ou le temps moyen passé dans l'état particulier par plus d'un dispositif médical portable est supérieur au le moment optimal consiste à fournir une indication sur un affichage.

6. Procédé mis en œuvre par ordinateur selon la revendication 1, dans lequel la surveillance du temps pendant lequel un ou plusieurs dispositifs médicaux portables se trouvent dans l'un des états du dispositif comprend le stockage du temps passé dans l'état d'un dispositif dans une mémoire associée à un dispositif de traitement.

7. Procédé mis en œuvre par ordinateur selon la revendication 1, dans lequel le temps optimal comprend une valeur de temps reçue du praticien.

8. Procédé mis en œuvre par ordinateur selon la revendication 1, dans lequel le temps optimal comprend une valeur de temps déterminée à partir d'une pluralité d'autres cycles de test.

9. Procédé mis en œuvre par ordinateur selon la revendication 1, dans lequel la fourniture d'une indication selon laquelle le temps passé dans l'état d'un dispositif par le dispositif médical portable ou le temps moyen passé dans l'état d'un dispositif par plus d'un dispositif médical portable est supérieur au moment optimal consiste à fournir une indication d'une action corrective.

10. Système (100) comprenant: un dispositif de traitement (102) auquel est associée une mémoire appropriée (104); et un dispositif d'entrée/sortie (106) structuré pour recevoir des informations d'un utilisateur et lui fournir des informations, dans lequel le dispositif de traitement est programmé pour: déterminer une pluralité d'états de dispositif au cours d'un cycle de test qui doivent être surveillés, au moins un des états de dispositif correspondant au moment où un dispositif médical portable est en possession d'un patient et au moins deux desdits états de dispositif correspondant au moment où le dispositif médical portable est en possession du praticien; surveiller le temps pendant lequel un ou plusieurs dispositifs médicaux portables se trouvent dans l'un des états du dispositif; déterminer que le temps passé dans l'état d'un dispositif par un dispositif médical portable ou le temps moyen passé dans l'état d'un dispositif par plusieurs dispositifs médicaux portables est supérieur à un temps optimal; et dans lequel la pluralité d'états de dispositif comprend un ou plusieurs des éléments suivants: un état correspondant au dispositif médical portable subissant un processus de recharge; et un état correspondant au dispositif médical portable subissant un processus de nettoyage, et le procédé consistant en outre à déterminer que le temps, ou le temps moyen, passé dans l'état correspondant au dispositif médical portable subissant un ou plusieurs processus de recharge et un processus de nettoyage par un ou plusieurs dispositifs médicaux portables est supérieur à un temps optimal, déterminer qu'une inefficacité existe dans l'utilisation dudit ou desdits dispositifs médicaux portables, et fournir une indication si le temps passé est supérieur au temps optimal.

11. Système selon la revendication 10, dans lequel le processeur est programmé pour déterminer la pluralité d'états de dispositif dans un cycle de test qui doivent être surveillés au moins en partie à partir d'une entrée reçue du dispositif d'entrée/sortie.

12. Système selon la revendication 10, dans lequel l'indication que le temps passé dans l'état de dispositif unique par le dispositif médical portable ou le temps moyen passé dans l'état de dispositif unique par plus d'un dispositif médical portable est supérieur au temps optimal est fournie dans un affichage sur le dispositif d'entrée/sortie.

13. Système selon la revendication 10, dans lequel le processeur est en outre programmé pour fournir une indication d'une action corrective.
